# FASCICULE DE BREVET EUROPEEN

(11) **EP 3 164 197 B1**
(45) Date de publication et mention de la délivrance du brevet: **09.09.2020**
(21) Numéro de dépôt: 15733764.3
(22) Date de dépôt: 03.07.2015
(51) Int. Cl.: A61Q 19/06, A61Q 19/08, A61K 8/9789

(54) **EXTRAITS PEPTIDIQUES DE LUPIN ET FERMETE DE LA PEAU**
PEPTIDISCHE EXTRAKTE AUS LUPINEN UND HAUTFESTIGKEIT
PEPTIDIC EXTRACTS OF LUPINE AND SKIN FIRMNESS

(30) Priorité: 03.07.2014 FR 1456385
(43) Date de publication de la demande: 10.05.2017
(73) Titulaire: Laboratoires Expanscience, 92048 Paris La Défense Cedex (FR)
(72) Inventeur: BAUDOUIN, Caroline, F-78120 Rambouillet (FR); LECLERE- BIENFAIT, Sophie, F-28100 Dreux (FR); MSIKA, Philippe, F-78000 Versailles (FR); BREDIF, Stéphanie, F-28210 Croisilles (FR)
(74) Mandataire: Regimbeau
(86) Numéro de dépôt international: PCT/EP2015/065254
(87) Numéro de publication internationale: WO 2016/001430

(56) Documents cités:
- WO-A1-02/15869
- FR-A1- 2 792 202
- FR-A1- 2 893 252
- US-A1- 2008 153 757
- US-A1- 2011 034 486
- DATABASE GNPD [Online] MINTEL; avril 2014 (2014-04), "Lotion", XP002736411, Database accession no. 2337946

## Description

### DOMAINE DE L'INVENTION

La présente invention décrit l'utilisation d'un extrait peptidique de lupin pour prévenir une perte de fermeté de la peau ou pour augmenter sa fermeté. Selon l'invention, l'extrait peptidique de lupin prévient l'apparition de capitons et/ou réduit les capitons de la peau.

### ARRIERE PLAN DE L'INVENTION

Le derme est une des trois couches constitutives de la peau comprise entre l'épiderme et l'hypoderme. Le derme est un tissu conjonctif de soutien de la peau, principalement composé d'une matrice extracellulaire (MEC) produite par les fibroblastes qui sont la principale population cellulaire dermique.

La matrice extracellulaire est composée de fibres protéiques (collagène et élastine) et d'une matrice extra-fibrillaire, également dénommée substance fondamentale, comprenant, entre autres, des glycoprotéines structurelles (fibronectine) et des protéoglycanes/ glycosaminoglycanes. Les collagènes représentent environ 70% des composants de la MEC, avec en majorité le collagène de type I (85-90% du collagène dermique) et le collagène de type III (10-15% du collagène dermique). Les collagènes confèrent fermeté au derme et résistance à la pression (résistance mécanique de la peau). Les fibres d'élastine lui donnent ses propriétés élastiques.

Si les collagènes sont essentiels au maintien de la fermeté de la peau, le rôle de la jonction épidermique (JDE) ne doit pas être négligé. La JDE est une structure complexe qui sépare le derme de l'épiderme. Ses principales fonctions sont celles de support mécanique pour l'adhésion de l'épiderme au derme et de barrière de diffusion et zone d'échanges entre les deux compartiments. L'adhésion de l'épiderme au derme est assurée entre autres par des protéines d'ancrage, telles que les collagènes de type IV ou la laminine 5. Lorsque le derme et l'épiderme sont moins solidaires, la peau perd de sa fermeté, l'épiderme se relâche et plisse.

La glycation est un phénomène bien connu au niveau de la peau, en particulier au niveau du derme. Il s'agit d'une réaction spontanée, non enzymatique, entre un groupement amine, en particulier un groupement amine d'un acide aminé constituant les protéines, par exemple la lysine, et un sucre réducteur, tel que le glucose ou le ribose.

Au niveau du derme, le glucose réagit tout particulièrement avec le collagène et/ou l'élastine, pour donner des composés à l'origine de produits dits « produits terminaux de glycation avancée », bien connus sous l'acronyme « AGEs ». Ces modifications chimiques ne sont pas sans conséquence sur les propriétés du collagène et/ ou de l'élastine. Par exemple, les AGEs induisent la formation de pontages moléculaires entre les fibres de collagène. Ces pontages altèrent les propriétés mécaniques des fibres de collagène en les rendant plus rigides.

Le phénomène de glycation peut ainsi affecter les protéines de la matrice extra-cellulaire dermique, comme le collagène, et ainsi altérer leurs propriétés métaboliques et mécaniques.

Au niveau de la peau, la glycation altérant l'organisation des fibres de collagène et les fonctionnalités des fibroblastes dermiques (comme la capacité de contraction dans des lattices de collagène), une perte de fermeté et d'élasticité des tissus est observée. La peau perd de sa fermeté, se relâche. La glycation des protéines, en particulier du collagène, entraîne donc des conséquences dommageables au niveau de la peau. Ces conséquences sont visiblement perceptibles et peuvent être plus ou moins inesthétiques. En particulier, en étant moins structuré, moins ferme, le tissu dermique résiste moins bien à la poussée des adipocytes situé dans l'hypoderme, créant en surface de la peau un aspect capitonné encore appelé « peau d'orange ».

L'art antérieur décrit une lotion anti-âge pour le visage et le cou comportant unhydrolysat de protéines de lupin en association avec d'autres ingrédients botaniques dans une lotion anti-âge (« Lotion », Database GNPD [online] MINTEL, avril 2014, database accession no. 2337946). La demande de brevet FR2893252 porte sur la stimulation de la synthèse de Hyaluronane Synthase par des extraits végétaux. La demande de brevet WO 02/15869 décrit des compositions cosmétiques comprenant un ou plusieurs principes actifs choisis parmi l'acide ursulique et oléanolique, le palmitoyl pentapeptide et un extrait peptidique de lupin blanc. La demande de brevet FR2792202 porte sur des extraits peptidiques de lupin inhibant l'activité des métalloprotéinases. La demande de brevet US 2008/153757 décrit une formulation topique destinée à rajeunir la peau ou ralentir le vieillissement photo-induit. La demande de brevet US 2011/034486 porte sur une préparation cosmétique ou dermatologique, comprenant des pterocarpanes combattant la cellulite.

Un besoin existe donc de trouver des agents permettant de prévenir une perte de fermeté de la peau ou d'augmenter sa fermeté. Avantageusement, un tel agent permettrait de prévenir l'apparition des capitons ou de réduire l'aspect capitonné de la peau, en particulier chez des sujets présentant de la cellulite.

### BREVE DESCRIPTION DE L'INVENTION

L'invention décrit l'utilisation d'un extrait peptidique de lupin pour prévenir une perte de fermeté de la peau ou pour augmenter sa fermeté, de préférence la peau du corps.

L'invention décrit également l'utilisation d'une composition cosmétique comprenant de 0.001 à 30% en poids sec d'un extrait peptidique de lupin pour prévenir une perte de fermeté de la peau ou pour augmenter sa fermeté, de préférence la peau du corps.

L'invention porte sur l'utilisation d'un extrait peptidique de lupin pour prévenir l'apparition de capitons ou réduire les capitons de la peau, ou pour remodeler la silhouette du corps, caractérisée en ce que l'extrait peptique de lupin est obtenu par hydrolyse enzymatique et ledit extrait peptidique comprend au moins 80% en poids de peptides par rapport au poids sec total de l'extrait.

L'invention porte également sur l'utilisation d'une composition cosmétique comprenant de 0.001 à 30% en poids sec, avantageusement de 0,05 à 0,2% en poids, d'un extrait peptidique de lupin pour prévenir l'apparition de capitons ou réduire les capitons de la peau, ou pour remodeler la silhouette du corps, caractérisée en ce que l'extrait peptique de lupin est obtenu par hydrolyse enzymatique et ledit extrait peptidique comprend au moins 80% en poids de peptides par rapport au poids sec total de l'extrait.

### BREVE DESCRIPTION DES DESSINS

Figures 1 et 2: Forces contractiles développées par les fibroblastes au sein d'un derme équivalent sous tension dans le système GlasBox®.
Figure 3 : Expression du Collagène de type I (Derme).
Figure 4 : Expression du Collagène de type III (Derme).
Figure 5 : Expression du Collagène de type IV (JDE).
Figure 6 : Expression de la laminine 5 (JDE).

### DESCRIPTION DETAILLEE DE L'INVENTION

Les extraits peptidiques de lupin sont connus pour présenter une activité anti-métalloprotéase, notamment anti-collagénase et anti-gélatinase. Sur la base de cette activité, il a été proposé de les utiliser pour traiter des maladies liées à une destruction excessive du collagène et/ou une destruction excessive des tissus de soutien. Par exemple, il a été proposé d'utiliser des extraits peptidiques de lupin pour traiter l'arthrose, les maladies parodontales, les lésions de la peau, les maladies inflammatoires, les maladies liées au défaut de cicatrisation, à l'attaque de l'émail des dents, à l'angiogénèse tumorale ou pathologique ou le traitement des liaisons de la peau dues au vieillissement intrinsèque de la peau, au vieillissement sous l'action du rayonnement solaire, aux effets délétères du tabac, de la pollution et du stress.

De manière inattendue, les inventeurs ont découvert que les extraits peptidiques de lupin présentent la propriété de diminuer voire inhiber la glycation des protéines, en particulier la glycation du collagène, de stimuler l'expression des collagènes I, III et IV, trois macromolécules importantes de la matrice extra-cellulaire dermique et de la jonction dermo-épidermique et de stimuler les forces contractiles des fibroblastes.
La présente invention tire donc avantage de ces nouvelles propriétés et décrit ainsi l'utilisation d'un extrait peptidique de lupin pour prévenir une perte de fermeté de la peau ou augmenter sa fermeté. Avantageusement, les extraits peptidiques de lupin selon l'invention sont utilisés en tant qu'agents anti-glycation.

En effet, en inhibant la glycation du collagène et en stimulant l'expression des collagènes I et III, les extraits peptidiques de lupin contribuent à renforcer la matrice extracellulaire. La matrice extracellulaire se trouvant renforcée, la peau gagne en fermeté. Par ailleurs, en stimulant la synthèse de collagène IV, les extraits peptidiques de lupin contribuent à renforcer l'adhésion du derme et de l'épiderme. Enfin, en protégeant le derme des pertes de contractions des fibres contractiles, les extraits peptidiques de lupin contribuent à renforcer la fermeté de la peau. Les extraits peptidiques de lupin sont donc particulièrement efficaces pour prévenir une perte de fermeté de la peau ou pour augmenter sa fermeté, tout particulièrement la fermeté de la peau du corps par opposition à celle du visage. La peau du corps est moins exposée aux agressions extérieures que celle du visage mais elle a aussi ses ennemis : la vie sédentaire, les variations de poids, les grossesses, les changements hormonaux... Ces phénomènes peuvent conduire à une perte de fermeté et de tonicité de la peau, engendrant l'apparition de désordres inesthétiques tels que relâchement cutané, capitons, cellulite.

Le terme cellulite se réfère à un désordre cutané localisé causé par la protrusion de graisses sous-cutanées dans le derme résultant en des altérations structurales et architecturales qui se caractérisent visuellement par des capitons irréguliers.

Les extraits peptidiques selon l'invention sont avantageusement utilisés en tant que produits de soin spécifiques pour le corps, en particulier comme produits amincissants, ou pour leur effet raffermissant, remodelant de la silhouette du corps, /tonifiant, anti-relâchement, anti-cellulite et/ou anti-capitons.

Par ailleurs, la matrice extracellulaire étant renforcée, le tissu dermique devient plus résistant à la poussée des adipocytes. L'apparition des capitons s'en trouve gênée. Les extraits peptidiques de lupin s'avèrent donc particulièrement efficaces pour prévenir l'apparition des capitons de la peau et/ou réduire les capitons de la peau.

Selon une étude de Kligman et al [J Dermatolog Treat, 1999 ; 10 :119-25] évaluant l'efficacité du rétinol sur la cellulite ; cette efficacité était principalement liée à la restructuration du derme et à l'augmentation de la micro-vascularisation.

Ainsi, une action sur le derme pour en renforcer la structure et promouvoir la fermeté cutanée semble primordiale pour améliorer l'apparence de la cellulite.

L'extrait peptidique de lupin utile dans le cadre de la présente invention comprend au moins 80% en poids de peptides par rapport au poids total sec de l'extrait peptidique, typiquement de 80 à 100% en poids de peptides par rapport au poids total sec de l'extrait peptidique.

L'extrait peptidique est essentiellement dépourvu de sucres et de lipides.

La teneur en sucre est typiquement inférieure à 4% en poids sec, de préférence comprise entre 0, voire 1, et 3%, en poids par rapport au poids total sec de l'extrait peptidique.

La teneur en lipides est typiquement inférieure à 1% en poids sec par rapport au poids total sec de l'extrait peptidique.

L'extrait peptidique de lupin peut être choisi parmi les extraits peptidiques issus de lupin appartenant au genre *lupinus albus, lupinus luteus, lupinus mutabilis* et leur mélange. De préférence, l'extrait peptidique est un extrait peptidique de lupin blanc doux (*lupinus albus*), par exemple de la variété Ares. Le lupin de la variété Ares présente une faible teneur en alcaloïdes.

L'extrait peptidique de lupin peut être obtenu par un procédé comprenant les étapes suivantes :
(a) extraction de la fraction protéique de lupin ;
(b) hydrolyse de la fraction protéique obtenue à l'étape (a) ;
(c) récupération d'un extrait peptidique.

L'hydrolyse est typiquement réalisée par hydrolyse enzymatique. En particulier, l'hydrolyse enzymatique peut être réalisée au moyen de protéases comme par exemple l'Alcalase® (NOVO NORDISK) ou la Prolyve 1000 (LYVEN). L'hydrolyse enzymatique peut être réalisée à une température allant de 50 à 60°C pendant un temps variant de 2 heures à 4 heures.

Les peptides de l'extrait peptidique possèdent avantageusement un poids moléculaire allant de 130 à 10000 Da, de préférence allant de 130 à 3500 Da, de manière encore préférée de 300 à 3500 Da.

Selon une caractéristique particulière de l'invention, 40 à 55% des peptides de l'extrait peptidique possèdent un poids moléculaire allant de 300 à 1200 Da, et/ou 25 à 40% des peptides dudit extrait peptidique possèdent un poids moléculaire allant de 1200 à 3500 Da.

L'extrait peptidique obtenu à l'étape (b) peut être purifié par diafiltration, de préférence sur un module d'ultrafiltration présentant un seuil de coupure allant de 10.000 daltons à 15 000 daltons. L'ultrafiltration permet d'éliminer les protéines. Ainsi, le procédé de préparation de l'extrait peptidique peut comprendre une étape d'ultrafiltration. Il peut également comprendre une étape de nanofiltration, par exemple sur un module de nanofiltration présentant un seuil de coupure allant de 100 à 300 Da, ou de 130 à 300 Da. La nanofiltration permet d'éliminer les acides aminés libres ou les sels minéraux.

Avant conditionnement, l'extrait peptidique peut être microfiltré (0,2 µm) de façon stérile puis:
- être réparti sous flux laminaire dans des contenants stériles sans conservateur ; ou
- être stabilisé microbiologiquement par ajout de conservateurs avant d'être répartie dans des contenants « propres » Les conservateurs utilisés pouvant être par exemple, un mélange de parabènes et/ou Phénoxyéthanol (comme le Phenonip à 0.5%). Un mélange sans parabènes sera privilégié comme par exemple le mélange Phénoxyéthanol (1%), acide citrique (0.5%), acide sorbique (0.08%) ; ou
- être stabilisé par séchage par atomisation ou lyophilisation afin d'obtenir un extrait peptidique sous forme sèche qui peut être conditionné dans des sachets hermétiques à l'abri de l'humidité et de contaminations microbiennes.

Ainsi, le procédé de préparation de l'extrait peptidique peut en outre comprendre une étape de concentration de l'extrait peptidique hydrolysé obtenu à l'étape (c) par évaporation partielle ou totale de l'eau.

Le séchage peut être réalisé par atomisation ou lyophilisation en présence ou non d'un support tel que la maltodextrine. De préférence, le séchage est réalisé par lyophilisation sans support.

L'extraction de la fraction protéique peut être réalisée de la manière suivante :
i) extraction des lipides de la graine de lupin au moyen d'un solvant approprié et récupération des fractions protéiques et saccharidiques du lupin ;
ii) extraction de la fraction protéique par ultrafiltration et récupération de ladite fraction protéique.

Le solvant utilisé à l'étape i) est avantageusement choisi dans le groupe des solvants alimentaires non toxiques, notamment l'éthanol. L'extraction des lipides est de préférence réalisée à partir de graines de lupin se présentant sous la forme d'un tourteau de lupin broyé ou d'une farine de lupin micronisée. L'extraction peut également être réalisée au moyen d'hexane.

Dans certains modes de réalisation, l'extrait peptidique de lupin présente la composition en acides aminés donnée dans le tableau A suivant (pourcentage en poids par rapport au poids total d'acides aminés) :

**Tableau A**

| aminoacides | % en poids par rapport au poids total d'acide animé |
|---|---|
| ASP | 11,3 |
| GLU | 23,2 |
| SER | 5,1 |
| HIS | 1,7 |
| GLY | 3,4 |
| THR | 3,2 |
| ALA | 2,8 |
| ARG | 10,3 |
| TIR | 6,1 |
| CYS-CYS | 2,4 |
| VAL | 3,8 |
| MET | 0,2 |
| PHE | 16,0 |
| ILE | 3,3 |
| LEU | 7,9 |
| LYS | 3,7 |
| PRO | 4,4 |

L'extrait peptidique de lupin est destiné à un usage cosmétique (non thérapeutique), de préférence en application topique sur la peau ou administré par voie orale.

L'extrait peptidique de lupin peut être formulé sous forme d'une composition cosmétique. Ainsi, la présente invention décrit également l'utilisation d'une composition cosmétique comprenant un extrait peptidique de lupin pour prévenir une perte de fermeté de la peau ou augmenter la fermeté de la peau, en particulier la peau du corps par opposition à la peau du visage. Selon la précédente invention, la composition cosmétique comprenant un extrait peptidique de lupin est utilisée pour prévenir l'apparition de capitons ou réduire les capitons de la peau, tout particulièrement chez les sujets présentant de la cellulite.

La composition cosmétique comprend de 0,001 à 30 %, de préférence de 0,01 à 5% en poids, de préférence de 0.05 à 5% en poids sec par rapport au poids total de la composition, d'un extrait peptidique de lupin. Avantageusement, la composition selon l'invention comprend de 0,01 à 0,2% en poids, encore plus avantageusement de 0,05 à 0,2% en poids, typiquement de 0,01 à 0,15% en poids, en particulier de 0,05 à 0,15% en poids, par rapport au poids total de la composition, d'un extrait peptidique de lupin. De telles concentrations peuvent constituer des concentrations efficaces d'extrait peptidique de lupin.

La composition cosmétique peut par ailleurs contenir des adjuvants habituels dans le domaine cosmétique, tels que des gélifiants, par exemple des gélifiants hydrophiles ou lipophiles, des conservateurs, des agents antioxydants, des solvants, des parfums, des charges, des filtres chimiques ou minéraux, des pigments, des agents chélateurs, des absorbeurs d'odeur, des eaux thermales et/ou des matières colorantes. Les quantités de ces différents adjuvants sont celles classiquement utilisées en cosmétique. Par exemple, la quantité de chaque adjuvant peut varier de 0,01% à 20% en poids, par rapport au poids total de la composition cosmétique.

La composition cosmétique peut en outre comprendre des agents actifs choisis parmi les actifs raffermissants, les actifs drainants, les actifs astringents, les actifs tenseurs ou leurs mélanges. La quantité individuelle de chaque agent actif peut varier de 0,01% à 20% en poids, par rapport au poids total de la composition cosmétique.

La composition cosmétique peut se présenter sous n'importe quelle forme usuellement utilisée dans le domaine cosmétique, en particulier sous n'importe quelle forme adaptée à une application externe topique. Ainsi la composition cosmétique peut se présenter sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, ou d'une dispersion d'huile dans une phase aqueuse à l'aide de sphérules, les sphérules pouvant être des nanoparticules polymériques, telles que les nanosphères et les nanocapsules, ou des vésicules lipidiques de type ionique et/ou non-ionique. La composition cosmétique peut être plus ou moins fluide, se présenter sous forme de crème, de pommade, de lait, de lotion, d'onguent, de sérum, de pâte, de mousse, d'aérosol ou de stick.

Lorsque la composition cosmétique est une émulsion, la proportion de la phase grasse peut aller de 5% à 80% en poids, de préférence de 5% à 50% en poids, par rapport au poids total de la composition cosmétique. Les huiles, les émulsionnants et les co-émulsionnants utilisés dans la composition cosmétique sont choisis parmi ceux classiquement utilisés dans le domaine cosmétique. L'émulsionnant et le co-émulsionnant peuvent être présents dans la composition en une proportion allant de 0,3% à 30% en poids, de préférence de 0,5% à 20% en poids, par rapport au poids total de la composition cosmétique. Les huiles peuvent être choisies parmi les huiles minérales, les huiles d'originale végétale (huile d'abricot, de tournesol, de prune), les huiles d'origine animale, les huiles de synthèse, les huiles siliconées et les huiles fluorées (perfluoropolyéthers). Des alcools gras, tels que l'alcool cétylique, des acides gras ou des cires, telles que la cire d'abeille, peuvent également être utilisées en tant que matières grasses. Les émulsionnants et coémulsionnants peuvent être choisis parmi les esters d'acide gras et de polyéthylène glycol, tels que le stéarate de PEG-40 ou le stéarate de PEG-100, les esters d'acide gras et de polyol, tels que le stéarate de glycérol et le tristéarate de sorbitan.

Dans certains modes de réalisation, la composition cosmétique peut être sous forme d'un gel. Un tel gel peut tout particulièrement être utile en tant que gel minceur anti-capitons. A titre d'exemple, un gel selon la présente invention peut présenter la composition suivante :

| | *% en poids par rapport au poids total de la composition* |
|---|---|
| EAU | QSP |
| ETHANOL | 1 à 50% |
| CAFFEINE | 1 à 10% |
| CARBOMER | 0 à 2% |
| AJUSTEUR pH | 0 à 2% |
| GLYCERINE | 1 à 5% |
| PEPTIDES LUPIN | 0.05 à 5% |
| CONSERVATEURS | 0 à 2% |
| | |

Dans certains modes de réalisation, la composition cosmétique peut être utile en tant que soin pour le corps raffermissant. A titre d'exemple, une telle composition peut présenter la composition suivante :

| | *% en poids par rapport au poids total de la composition* |
|---|---|
| EAU | QSP |
| ETHANOL | 1 à 50% |
| CAFFEINE | 1 à 10% |
| EMULSIONNANTS | 1 à 10% |
| AJUSTEUR pH | 0 à 2% |
| GLYCERINE | 1 à 5% |
| PEPTIDES LUPIN | 0.05 à 5% |
| CONSERVATEURS | 0 à 2% |
| HUILE | 5 à 20% |
| GOMME XANTHANE | 0 à 2% |
| CETYL ALCOHOL | 0 à 2% |

Il est décrit que l'extrait peptidique de lupin et la composition cosmétique comprenant un tel extrait peuvent être utilisés pour prévenir une perte de fermeté de la peau ou pour augmenter sa fermeté. Ainsi, il est décrit que l'extrait peptidique de lupin ou la composition cosmétique comprenant un tel extrait peut être utilisé dans une méthode de traitement cosmétique pour prévenir une perte de fermeté ou pour augmenter la fermeté de la peau comprenant l'application sur la peau, en particulier la peau du corps, d'un extrait peptidique de lupin, en particulier d'une concentration efficace d'un extrait peptidique de lupin, ou d'une composition comprenant un tel extrait.

Selon l'invention, l'extrait peptidique de lupin ou la composition cosmétique comprenant un tel extrait est utilisé dans une méthode de traitement cosmétique pour raffermir le corps comprenant l'application sur la peau d'un extrait peptidique de lupin, en particulier d'une concentration efficace d'un extrait peptidique de lupin, ou d'une composition comprenant un tel extrait. Le raffermissement obtenu permet un remodelage de la silhouette.

Selon l'invention, l'extrait peptidique de lupin et la composition cosmétique comprenant un tel extrait sont utilisés pour prévenir l'apparition de capitons et/ou réduire l'aspect capitonné de la peau. En d'autres termes, l'extrait peptidique de lupin permet de réduire l'aspect « peau d'orange » que peut présenter la peau de sujets présentant de la cellulite. La peau s'en trouve lissée. Ainsi, l'extrait peptidique de lupin ou la composition cosmétique comprenant un tel extrait peut être utilisé dans une méthode de traitement cosmétique pour prévenir l'apparition de capitons et/ou réduire l'aspect capitonné de la peau comprenant l'application sur la peau, en particulier la peau du corps, d'un extrait peptidique de lupin, en particulier d'une concentration efficace d'un extrait peptidique de lupin, ou d'une composition comprenant un tel extrait.

Les méthodes de traitement cosmétique telles que décrites ci-dessus font également l'objet de la présente invention.

### EXEMPLES

L'extrait peptidique utilisé dans les exemples ci-dessous est tel que fourni par les Laboratoires Expanscience sous la référence commerciale ACTIMP® (poudre) ou ACTIMP® 1.9.3 (liquide).

Il peut être préparé de la manière suivante.

### 1. Préparation des extraits peptidiques de lupin

### Préparation d'un tourteau de lupin délipidé et broyé

Les graines de lupin sont broyées et les lipides extraits au moyen d'éthanol.

### Extraction et purification des protéines de lupin

Cette étape comprend une solubilisation aqueuse de la fraction soluble à pH alcalin suivie d'une séparation des insolubles.

A partir du tourteau de lupin délipidé et broyé, l'extraction des protéines est réalisée à pH 9,0 (pH ajusté par ajout de soude) avec un ratio farine/eau égal à 1/10 (p/p) La solution est incubée sous agitation à température ambiante pendant une heure. La partie insoluble du tourteau est ensuite séparée de la partie soluble par essorage. Le tourteau obtenu est lavé. La fraction soluble contenant les protéines et les sucres solubles est diafiltrée sur un module d'ultrafiltration de seuil de coupure de 10.000 daltons afin de séparer les protéines (rétentat) des sucres solubles (ultrafiltrat).

### Production et purification de peptides par hydrolyse enzymatique :

Le rétentat d'ultrafiltration contenant les protéines est ajusté à la concentration de 100 g/1 puis hydrolysé à pH 8,0 en présence d'Alcalase# (NOVO NORDISK) à 55° C pendant 3 h environ. Après hydrolyse, l'enzyme est dénaturée par hydrolyse pendant 15 min à 85°C. Dès que la solution est refroidie, elle est neutralisée par ajout d'acide chlorhydrique. Les peptides obtenus sont purifiés par diafiltration sur module d'ultrafiltration de seuil de coupure de 10 000 daltons. La solution obtenue est ensuite nanofiltrée afin de dessaler (élimination du chlorure de sodium) et de concentrer la fraction peptidique. La solution de peptides est enfin décolorée à l'aide de charbon actif 3 % (1 heure à 50°C), le charbon étant éliminé par filtration.

### Stérilisation et stabilisation de la fraction de peptides:

Avant conditionnement, la solution est microfiltrée (0,2 µm) de façon stérile puis elle peut :
- être stabilisée microbiologiquement par ajout d'un mélange sans parabènes (Phénoxyéthanol (1%), acide citrique (0.5%), acide sorbique (0.08%)) avant d'être répartie dans des contenants « propres » (Actimp®1.9.3 (constitué de 10% de peptides de lupin), commercialisé par les Laboratoires Expanscience)
- être stabilisée par séchage par lyophilisation afin d'obtenir les peptides sous forme sèche, conditionnée dans des sachets hermétiques à l'abri de l'humidité et de contaminations microbiennes (Actimp® poudre, commercialisé par les Laboratoires Expanscience).

L'extrait sec et donc la partie active présente les caractéristiques suivantes :
- Aspect : poudre homogène non hygroscopique
- Couleur : blanc cassé

Composition chimique :
- Teneur en sucres totaux (dosage à l'anthrone) : < 4 %
- Teneur en chlorures (Kit SIGMA réf : 955-30) <6 %
- Teneur en eau (100° C, 4 h) < 8 %
- Teneur en peptides : ≥ 80%
   - pH (solution à 20 g/l) : 7,06
   - Solubilité (eau osmosée) : >100 g/L

**Tableau 1 - Composition en acides aminés de l'hydrolysat**

| Amino-acides | P.M. A.A. | Conc. en mM | Conc. en mg/l | % dans poudre | %/AA totaux |
|---|---|---|---|---|---|
| ASP | 133,1 | 2,078 | 276,582 | 9,9 | 11,3 |
| GLU | 147,1 | 3,858 | 567,438 | 20,3 | 23,2 |
| SER | 105,1 | 1,196 | 125,647 | 4,5 | 5,1 |
| HIS | 155,2 | 0,270 | 41,904 | 1,5 | 1,7 |
| GLY | 75,1 | 1,114 | 83,624 | 3,0 | 3,4 |
| THR | 119,1 | 0,664 | 79,023 | 2,8 | 3,2 |
| ALA | 89,1 | 0,763 | 67,983 | 2,4 | 2,8 |
| ARG | 174,2 | 1,447 | 251,980 | 9,0 | 10,3 |
| TYR | 181,2 | 0,829 | 150,215 | 5,4 | 6,1 |
| CYS-CYS | 240,3 | 0,247 | 59,234 | 2,1 | 2,4 |
| VAL | 117,1 | 0,792 | 92,743 | 3,3 | 3,8 |
| MET | 149,2 | 0,029 | 4,327 | 0,2 | 0,2 |
| PHE | 165,2 | 1,044 | 172,469 | 6,2 | 7,0 |
| ILE | 131,2 | 0,621 | 81,410 | 2,9 | 3,3 |
| LEU | 131,2 | 1,481 | 194,307 | 6,9 | 7,9 |
| LYS | 146,2 | 0,626 | 91,448 | 3,3 | 3,7 |
| PRO | 115,1 | 0,935 | 107,619 | 3,8 | 4,4 |
| | | 2447,952 | | | |
| | | Total | | 87,4 % | |

Dans les tests décrits ci-dessus, l'extrait peptidique est solubilisé dans le milieu de culture de façon à être appliqué sur les cellules et explants à des concentrations de 0.1%, 0.2% et/ou 2%.

### 2. Mise en évidence des effets de l'extrait peptidique de lupin sur la glycation

L'introduction non enzymatique des AGEs dans des fibres de collagène synthétisées et déposées par des fibroblastes dermiques humains cultivés *in vitro* a été étudiée. Une méthode de détection radioactive, reposant sur l'incorporation de glucose tritié dans les protéines de la matrice extracellulaire, a été utilisée.

Des fibroblastes dermiques humains normaux ont été cultivés en présence de vitamine C afin de stimuler la synthèse de collagène. Les cellules ont ensuite subi différentes opérations (congélation/décongélation/chauffage) visant à inactiver toute activité enzymatique.

Les tapis de matrice ainsi obtenus ont été traités en présence de l'extrait peptidique de lupin décrit ci-dessus à 0,2 et 2%, d'aminoguanidine HCl à 1 mg/ml (référence), de glucose à 0,2% et du marqueur radioactif [³H]-glucose pendant 15 jours à 37°C, en absence d'oxygène (une réoxygénation rapide a été effectuée après 7 jours de traitement).

A la fin de l'incubation, les protéines de la matrice extra-cellulaire (principalement des collagènes) ont été extraites à l'aide d'un tampon chaotropique, précipitées par de l'acide trichloroacétique puis collectées sur filtres. La radioactivité incorporée dans les fibres de la MEC a été mesurée par scintillation liquide.

Les résultats ont été analysés statistiquement au moyen d'une analyse de variance à un facteur suivie d'un test de Dunnett et sont présentés dans le tableau ci-dessous :

**Effet sur la glycation non enzymatique de [³H]-glucose**

| | Incorporation [³H]-glucose (cpm) | Inhibition de la glycation |
|---|---|---|
| Contrôle | 5527 ± 464 | |
| Aminoguanidine 1 mg/ml | 1219 ± 92 | -78% p<0,001 |
| Extrait peptidique de lupin 0,2% | 4466 ± 655 | -19% ns |
| Extrait peptidique de lupin 2% | 3338 ± 465 | -40% p<0,05 |

Les résultats montrent un effet protecteur de l'extrait peptidique de lupin vis-à-vis de la glycation.

L'extrait peptidique de lupin protège la matrice extra-cellulaire du derme de la glycation. L'extrait peptidique de lupin est donc capable d'empêcher la rigidification de la matrice pour préserver l'élasticité et la fermeté de la peau (*protection des propriétés biomécaniques du collagène).*

### 3. Mise en évidence des effets de l'extrait peptidique de lupin sur les forces contractiles

Le modèle de dermes équivalents, contenant des fibroblastes, permet d'évaluer la qualité des interactions entre des fibroblastes et les fibres de collagène. En effet, la contraction/ rétraction libre de ces dermes équivalents résulte de l'activité dynamique des cellules qui exercent des tractions sur le réseau de collagène.

L'effet de l'extrait peptidique de lupin sur la contraction de dermes équivalents a été évalué. Afin d'induire une condition de perte des forces de contraction des fibroblastes, les dermes équivalents ont été ensemencés par des fibroblastes sénescents, dits « âgés » (obtenus par repiquages successifs selon le modèle de Hayflick).

Les dermes équivalents « âgés » ou normaux ont été préparés par mélange de fibroblastes (à passage 8 pour les dermes équivalents normaux ou à passage 17 pour les dermes équivalents « âgés ») et d'une solution de collagène.
Après gélation complète, les dermes équivalents ont été traités par le TGFβ à 10 ng/ml (référence) ou l'extrait peptidique de lupin à 0,1% puis incubés pendant 4 jours.
Les dermes équivalents ont été photographiés à J1, J3 et J4 afin de mesurer leur surface, qui rend compte de la contraction.

Les résultats ont été analysés statistiquement à l'aide d'un test t de Student et sont présentés dans le tableau ci-dessous.

**Evolution de la contraction de dermes équivalents normaux ou « âgés »**

| | | **Contrôle (DE normaux)** | **Témoin (DE « âgés »)** | **TGFβ 10 ng/ml** | **Extrait peptidique de lupin 0,1%** |
|---|---|---|---|---|---|
| **J1** | Aire en mm² (moyenne ± sem) | 284,9 ± 2,9 | 282,7 ± 6,2 | 264,6 ± 5,5 | **243,1 ± 3,9** |
| | % du témoin « âgés » | 101 | 100 | 94 | **86 (p<0,01)** |
| **J3** | Aire en mm² (moyenne ± | 283,6 ± 11,6 | 300,1 ± 13,1 | 285,9 ± 6,7 | **248,5 ± 1,5** |
| | sem) | | | | |
| | *%* du témoin « âgés » | 95 | 100 | 95 | **83 (p<0,05)** |
| **J4** | Aire en mm² (moyenne ± sem) | 205,0 ± 5,3 | 237,7 ± 2,6 | 201,3 ± 8,4 | **217,5 ± 2,7** |
| | *%* du témoin « âgés » | 86 (p<0,01) | 100 | 85 (p<0,05) | **92 (p<0,01)** |

Globalement, la contraction des dermes équivalents (DE) « âgés » est plus faible que celle des DE normaux (l'aire des DE « âgés » est plus élevée), ceci reflète la diminution de l'interaction fibroblastes / fibres de collagène induite par le vieillissement des cellules.

Le TGFβ stimule la contraction des DE « âgés », cette stimulation devient significative après 4 jours de traitement ; ce résultat valide l'essai.

L'extrait peptidique de lupin à 0,1% stimule significativement la contraction des DE « âgés » dès le 1er jour de traitement.
Il permet de contrer précocément l'effet du vieillissement sur le « relâchement » des dermes équivalents.

L'extrait peptidique de lupin inhibe le « relâchement » des dermes équivalents induits ici par le vieillissement des cellules.
En protégeant le derme contre la perte de contraction, l'extrait peptidique de lupin contribue à maintenir les propriétés élastiques de la peau, pour un effet « tenseur » et un maintien de sa fermeté.

### 4. Mise en évidence des effets de l'extrait peptidique de lupin sur les forces isométriques

L'effet de l'extrait peptidique de lupin a été recherché sur les forces isométriques développées par des fibroblastes dermiques dans un derme équivalent sous tension (modèle GlasBox®).

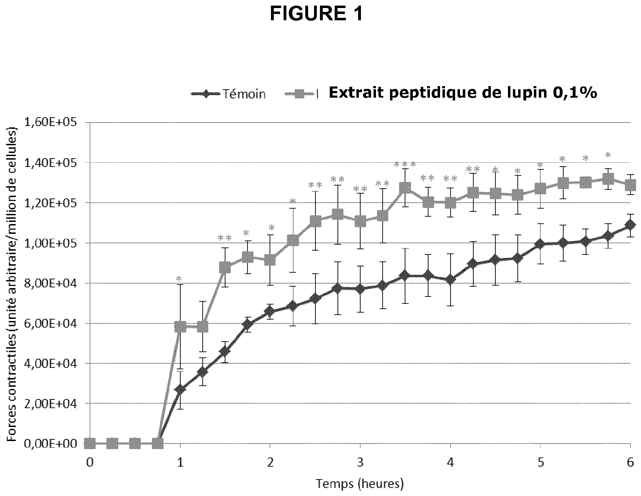

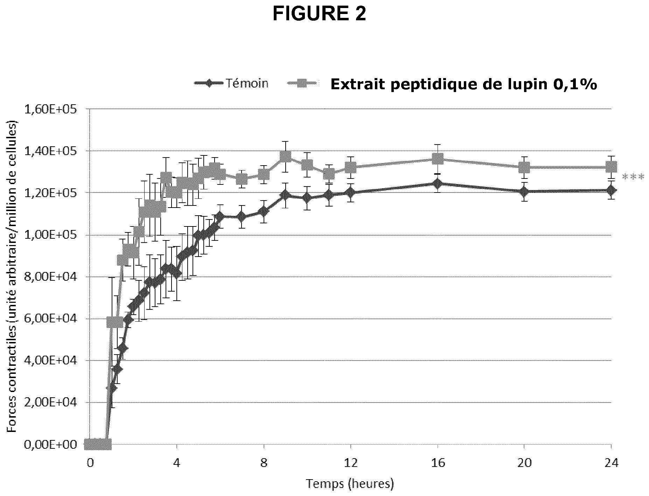

Le système GlasBox® (Growing LAttice Study BOX) permet de mesurer, au sein d'un derme équivalent sous tension, les forces contractiles développées par les fibroblastes. Celles-ci témoignent de l'activité des fibroblastes pour réorganiser la matrice.
Dans le système GlasBox®, les dermes équivalents se développent sous tension entre deux lames qui servent également de capteurs : sous l'influence de la force de rétraction développée par des fibroblastes, les lames se déforment. Cela se traduit par une variation de la résistance électrique de la jauge de contrainte. Cette variation, indicative de la force développée au sein du derme équivalent, est mesurée en temps réel.
Les dermes équivalents sont préparés en mélangeant des fibroblastes dermiques humains normaux à une solution de collagène. Ce mélange est coulé dans les cuves rectangulaires de la GlasBox®.
Après constitution d'un gel, un milieu contenant ou non l'extrait peptidique de lupin à 0,1% est ajouté.
Les forces isométriques sont mesurées pendant 24 heures.
Les résultats ont été analysés statistiquement au moyen d'une analyse de variance à deux facteurs suivie d'un test de Fisher et sont présentés aux figures 1 et 2.

De façon globale, l'extrait peptidique de lupin à 0,1% augmente significativement les forces développées par les fibroblastes. Cette augmentation est plus particulièrement significative dans la première partie de la courbe (après 1 heure et jusqu'à 5,75 heures de mesure).
Ceci montre un effet tenseur de l'extrait peptidique de lupin en particulier sur la phase précoce de réorganisation de la matrice par les fibroblastes.
Les forces développées par des fibroblastes au sein d'un derme équivalent sous tension sont significativement stimulées par l'extrait peptidique de lupin. Ces résultats mettent en évidence un effet tenseur de l'extrait peptidique de lupin pour lutter contre la perte de fermeté et d'élasticité de la peau.

### 5. Mise en évidence des effets de l'extrait peptidique de lupin sur l'expression des collagènes I, III et IV ainsi que de la laminine 5

L'activité de l'extrait peptidique de lupin a été évaluée sur des explants de peau humaine : des marqueurs de la matrice extracellulaire dermique (Collagènes I et III) et de la jonction dermo-épidermique (Collagène IV, laminine 5) ont été analysés par immunomarquage.

L'extrait peptidique de lupin à 0,2% a été appliqué en surface d'explants de peau normale humaine maintenus en survie.

Après 7 et 10 jours de traitement, les explants cutanés ont été prélevés et congelés à -80°C ou fixés puis inclus en paraffine.

Des immunomarquages de différents marqueurs de la matrice dermique ou de la jonction dermo-épidermique ont été réalisés sur les coupes de peau :
Les collagènes I et IV, ainsi que la laminine 5, ont été marqués sur coupes congelées et révélés en fluorescence (FITC), les noyaux ont été contre-colorés à l'iodure de propidium ;
Le collagène III a été marqué sur coupe paraffine et révélé à la peroxydase.

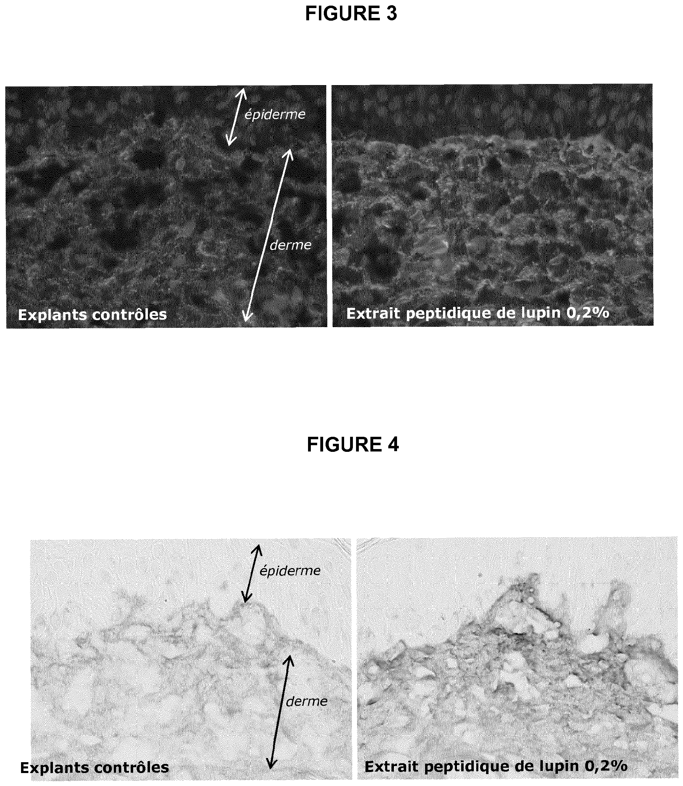

Les marquages ont été visualisés en microscopie et quantifiés par analyse d'image.
Les variations d'intensité de marquage ont été analysées statistiquement par un test t de Student.
Les résultats sont présentés aux figures 3 à 5.

L'extrait peptidique de lupin induit une intensification du marquage du collagène I après 7 jours et 10 jours de traitement (figure 3).

| | **Collagène I** (% de surface) | | | |
|---|---|---|---|---|
| | J7 | | J10 | |
| Explants contrôles | 28,0 ± 7,2 | | 21,9 ± 6,4 | |
| Extrait peptidique de lupin 0,2% | 37,7 ± 7,6 | **+34% p<0,05** | 44,2 ± 6,8 | **+102% p<0,001** |

### Quantification en analyse d'image du marquage du Collagène I dans le derme papillaire

L'extrait peptidique de lupin induit une intensification du marquage du collagène III après 7 et 10 jours de traitement (figure 4).

| | **Collagène III** (% de surface) | | | |
|---|---|---|---|---|
| | J7 | | J10 | |
| Explants contrôles | 30,9 ± 12,2 | | 28,3 ± 8,1 | |
| Extrait peptidique de lupin 0,2% | 51,3 ± 12,4 | **+66% p<0,01** | 49,8 ± 9,6 | **+76% p<0,001** |

### Quantification en analyse d'image du marquage du Collagène III dans le derme papillaire

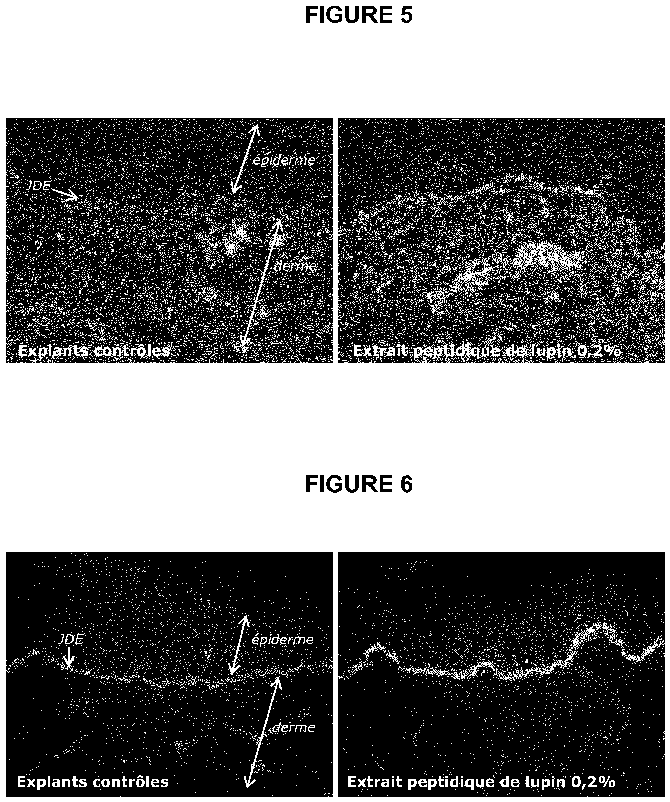

L'extrait peptidique de lupin induit une intensification du marquage du collagène IV au niveau de la JDE après 7 jours et 10 jours de traitement (figure 5).

| | **Collagène IV** (% de surface) | | | |
|---|---|---|---|---|
| | J7 | | J10 | |
| Explants contrôles | 22,0 ± 3,5 | | 14,1 ± 4,0 | |
| Extrait peptidique de lupin 0,2% | 29,8 ± 8,3 | **+35% p<0,05** | 44,9 ± 14,5 | **+219% p<0,001** |

### Quantification en analyse d'image du marquage du Collagène IV dans la JDE

L'extrait peptidique de lupin induit une intensification du marquage de la laminine 5 au niveau de la JDE après 7 jours de traitement (figure 6).

| | Laminine 5 (% de surface) à J7 | |
|---|---|---|
| Explants contrôles | 18,4 ± 5,1 | |
| Peptides de lupin 0,2% | 24,2 ± 4,4 | **+31% p<0,05** |

### Quantification en analyse d'image du marquage de la laminine 5 dans la JDE

L'application topique de l'extrait peptidique de lupin sur des explants cutanés a stimulé l'expression des collagènes I et III, deux macromolécules importantes de la matrice extra-cellulaire dermique et stimulé l'expression du collagène IV et de la laminine 5, marqueurs de la jonction dermo-épidermique.

Ainsi l'extrait peptidique de lupin est capable de renforcer la jonction dermo-épidermique et la matrice extracellulaire dermique pour favoriser les propriétés de fermeté et de tonicité cutanée.

## Revendications

1. Utilisation d'un extrait peptidique de lupin pour prévenir l'apparition de capitons ou réduire les capitons de la peau, ou pour remodeler la silhouette du corps, **caractérisée en ce que** l'extrait peptidique de lupin est obtenu par hydrolyse enzymatique et ledit extrait peptidique comprend au moins 80% en poids de peptides par rapport au poids sec total de l'extrait.

2. Utilisation selon l'une des revendications précédentes dans laquelle les peptides dudit extrait peptidique de lupin ont un poids moléculaire inférieur à 10 000Da.

3. Utilisation selon l'une des revendications précédentes dans laquelle l'extrait peptidique comprend moins de 4% en poids de sucres et/ou comprend moins de 1% en poids de lipides.

4. Utilisation selon l'une des revendications précédentes dans laquelle les peptides de l'extrait peptidique ont un poids moléculaire supérieur à 130 Da.

5. Utilisation selon l'une des revendications précédentes dans laquelle l'extrait peptidique est un extrait peptidique de *lupinus albus.*

6. Utilisation selon l'une des revendications précédentes dans laquelle l'extrait peptidique de lupin est susceptible d'être obtenu par un procédé comprenant les étapes suivantes :
(a) extraction de la fraction protéique de lupin ;
(b) hydrolyse enzymatique de la fraction protéique obtenue à l'étape (a);
(c) récupération de l'extrait peptidique hydrolysé.

7. Utilisation selon l'une des revendications précédentes en application topique ou administré par voie orale.

8. Utilisation d'une composition cosmétique comprenant de 0.001 à 30% en poids sec, avantageusement de 0,05 à 0,2% en poids, d'un extrait peptidique de lupin pour prévenir l'apparition de capitons ou réduire les capitons de la peau, ou pour remodeler la silhouette du corps, **caractérisée en ce que** l'extrait peptique de lupin est obtenu par hydrolyse enzymatique et ledit extrait peptidique comprend au moins 80% en poids de peptides par rapport au poids sec total de l'extrait.

9. Utilisation selon la revendication 8 dans laquelle la composition cosmétique comprend en outre des gélifiants, des conservateurs, des agents antioxydants, des solvants, des parfums, des charges, des filtres chimiques ou minéraux, des pigments, des agents chélateurs, des absorbeurs d'odeur, des eaux thermales et/ou des matières colorantes.

## Patentansprüche

1. Verwendung eines Peptidextrakts von Lupine zur Vorbeugung von Fettpölsterchen oder Reduzierung von Fettpölsterchen oder zur Verbesserung der Silhouette des Körpers, **dadurch gekennzeichnet, dass** der Peptidextrakt von Lupine durch enzymatische Hydrolyse erhalten wird und das der Peptidextrakt mindestens 80 Gew.-% Peptide in Bezug auf das Trockengewicht insgesamt des Extrakts umfasst.

2. Verwendung nach einem der vorangehenden Ansprüche, wobei die Peptide des Peptidextrakts von Lupine ein Molekulargewicht unter 10.000 Da haben.

3. Verwendung nach einem der vorangehenden Ansprüche, wobei der Peptidextrakt weniger als 4 Gew.-% Zucker umfasst und/oder weniger als 1 Gew.-% Lipide umfasst.

4. Verwendung nach einem der vorangehenden Ansprüche, wobei die Peptide des Peptidextrakts ein Molekulargewicht über 130 Da haben.

5. Verwendung nach einem der vorangehenden Ansprüche, wobei der Peptidextrakt ein Peptidextrakt von *lupinus albus* ist.

6. Verwendung nach einem der vorangehenden Ansprüche, wobei der Peptidextrakt von Lupine durch ein Verfahren gewinnbar ist, das die folgenden Schritte umfasst:
(a) Extrahieren der Proteinfraktion von Lupine;
(b) enzymatisches Hydrolysieren der in Schritt (a) erhaltenen Proteinfraktion;
(c) Rückgewinnen des hydrolysierten Peptidextrakts.

7. Verwendung nach einem der vorangehenden Ansprüche in topischer Auftragung oder verabreicht auf oralem Weg.

8. Verwendung einer kosmetischen Zusammensetzung, umfassend 0,001 bis 30 Gew.-% trocken, in vorteilhafter Weise 0,05 bis 0,2 Gew.-%, eines Peptidextrakts von Lupine zur Vorbeugung von Fettpölsterchen oder Reduzierung von Fettpölsterchen oder zur Verbesserung der Silhouette des Körpers, **dadurch gekennzeichnet, dass** der Peptidextrakt von Lupine durch enzymatische Hydrolyse erhalten wird und der Peptidextrakt mindestens 80 Gew.-% Peptide in Bezug auf das Trockengewicht insgesamt des Extrakts umfasst.

9. Verwendung nach Anspruch 8, wobei die kosmetische Zusammensetzung ferner Geliermittel, Konservierungsstoffe, Antioxidationsmittel, Lösungsmittel, Duftstoffe, Zuschläge, chemische oder mineralische Filter, Pigmente, Chelatbildner, Geruchsabsorber, Thermalwasser und/oder colorierende Stoffe umfasst.

## Claims

1. Use of a lupin peptide extract to prevent the onset of skin dimpling or to reduce skin dimpling, or to remodel the body silhouette, **characterized in that** the lupin peptide extract is obtained by enzymatic hydrolysis and said peptide extract comprises at least 80% by weight of peptides relative to the total dry weight of the extract.

2. The use according to one of the preceding claims, wherein the peptides of said lupin peptide extract have a molecular weight of less than 10,000 Da.

3. The use according to one of the preceding claims, wherein the peptide extract comprises less than 4% by weight of sugars and/or comprises less than 1% by weight of lipids.

4. The use according to one of the preceding claims, wherein the peptides of the peptide extract have a molecular weight higher than 130 Da.

5. The use according to one of the preceding claims, wherein the peptide extract is a peptide extract of *Lupinus albus.*

6. The use according to one of the preceding claims, wherein the lupin peptide extract is obtainable by a method comprising the following steps:
(a) extracting the lupin protein fraction;
(b) enzymatically hydrolysing the protein fraction obtained in step (a);
(c) recovering the hydrolysed peptide extract.

7. The use according to one of the preceding claims in topical application or taken via oral route.

8. Use of a cosmetic composition comprising 0.001 to 30% by dry weight, advantageously 0.05 to 0.2% by weight, of a lupin peptide extract to prevent the onset of skin dimpling or to reduce skin dimpling, or to remodel the body silhouette, **characterized in that** the lupin peptide extract is obtained by enzymatic hydrolysis and said peptide extract comprises at least 80% by weight of peptides relative to the total dry weight of the extract.

9. The use according to claim 8, wherein the cosmetic composition further comprises gelling agents, preserving agents, antioxidants, solvents, perfumes, fillers, chemical or mineral filters, pigments, chelating agents, odour absorbers, thermal water and/or colouring materials.
